# EUROPEAN PATENT APPLICATION

(11) **EP 1 256 326 A1**
(43) Date of publication of application: **13.11.2002**
(21) Application number: 02251389.9
(22) Date of filing: 27.02.2002
(51) Int. Cl.: A61B 18/14

(54) **Catheter having continuous braided electrode**

(30) Priority: 08.05.2001 US 796198
(71) Applicant: Biosense Webster, Inc., Diamond Bar, California 91765 (US)
(72) Inventor: Mest, Robert A., Long Beach, California 90808 (US); Hill, Irma, La Verne, California 91750 (US)
(74) Representative: Mercer, Christopher Paul

(57) **Abstract**

A catheter for ablating tissue comprises an elongated flexible catheter body having a continuous electrode comprising a braided conductive mesh surrounding a flexible plastic tubing. In a preferred embodiment, the catheter comprises a catheter body, a tip section at the distal end of the catheter body, and an electrode assembly at the distal end of the tip section. The electrode assembly comprises a flexible plastic tubing having an outer wall with a plurality of irrigation holes extending therethrough, a generally circular main region that is generally transverse to the axis of the catheter body, a continuous electrode formed of a braided conductive mesh surrounding the flexible plastic tubing and extending over substantially all of the generally circular main region, and a support member having shape memory extending within a lumen of the flexible plastic tubing. An electrode lead wire electrically connects the continuous electrode to a source of ablation energy. The catheter further comprises means for introducing an irrigation fluid into a lumen of the flexible plastic tubing of the electrode assembly so that the fluid can pass out of the electrode assembly through the irrigation holes.

## Description

### FIELD OF THE INVENTION

The present invention relates to a catheter having a continuous braided electrode useful for creating linear lesions.

### BACKGROUND OF THE INVENTION

Atrial fibrillation is a common sustained cardiac arrhythmia and a major cause of stroke. This condition is perpetuated by reentrant wavelets propagating in an abnormal atrial-tissue substrate. Various approaches have been developed to interrupt wavelets, including surgical or catheter-mediated atriotomy. It is believed that to treat atrial fibrillation by radio-frequency ablation using a catheter, continuous linear lesions must be formed to segment the heart tissue. By segmenting the heart tissue, no electrical activity can be transmitted from one segment to another. Preferably, the segments are made too small to be able to sustain the fibrillatory process. A preferred approach for treating atrial fibrillation is by forming a linear lesion where a relatively long electrode can be held stationary in good contact with the heart wall while ablation is completed. In this way, a continuous transmural burn may be effected.

### SUMMARY OF THE INVENTION

The invention is directed to a catheter for ablating tissue comprises an elongated flexible catheter body having a continuous electrode comprising a braided conductive mesh surrounding a flexible plastic tubing.

In one embodiment, the catheter comprises a catheter body having an outer wall, proximal and distal ends, and at least one lumen extending therethrough. A tip section comprising a segment of flexible tubing having proximal and distal ends and at least one lumen therethrough is fixedly attached to the distal end of the catheter body. An electrode assembly is provided at the distal end of the tip section. The electrode assembly comprises a flexible plastic tubing and a continuous electrode formed of a braided conductive mesh surrounding the flexible plastic tubing. An electrode lead wire electrically connects the continuous electrode to a source of ablation energy.

In a particularly preferred embodiment, the catheter comprises a catheter body, a tip section at the distal end of the catheter body, and an electrode assembly at the distal end of the tip section. The electrode assembly comprises a flexible plastic tubing having an outer wall with a plurality of irrigation holes extending therethrough, a generally circular main region that is generally transverse to the axis of the catheter body, a continuous electrode formed of a braided conductive mesh surrounding the flexible plastic tubing and extending over substantially all of the generally circular main region, and a support member having shape memory extending within a lumen ofthe flexible plastic tubing. An electrode lead wire electrically connects the continuous electrode to a source of ablation energy. The catheter further comprises means for introducing an irrigation fluid into a lumen of the flexible plastic tubing of the electrode assembly so that, in use, the fluid can pass out of the electrode assembly through the irrigation holes.

### DESCRIPTION OF THE DRAWINGS

These and other features and advantages of the present invention will be better understood by reference to the following detailed description when considered in conjunction with the accompanying drawings wherein:
FIG. 1 is a side cross-sectional view of an embodiment of the catheter of the invention.
FIG. 2 is a side cross-sectional view of a catheter body according to the invention, including the junction between the catheter body and tip section.
FIG. 3 is a schematic perspective view of the electrode assembly according to the invention.
FIG. 4 is a side view of the electrode assembly according to the invention in a clockwise formation.
FIG. 5 is a side view of the electrode assembly according to the invention in a counterclockwise formation rotated 90° relative to the assembly depicted in FIG. 4.
FIG. 6 is a side view of the plastic tubing used to form the electrode assembly in a straight configuration.
FIG. 7A is a side view of the electrode assembly in a straight configuration and also shows the junction of the electrode assembly and the tip section.
FIG. 7B is an enlarged view of a portion of the proximal end of the electrode assembly depicted in FIG. 7A.
FIG. 7C is an enlarged view of a portion of the distal end of the electrode assembly depicted in FIG. 7A.
FIG. 8 is a cross sectional view of a portion of the catheter tip section showing one means for attaching the puller wire.
FIG. 9 is a top cross sectional views of a preferred puller wire anchor.
FIG. 10 is a side cross sectional views of the puller wire anchor of FIG. 9.

### DETAILED DESCRIPTION

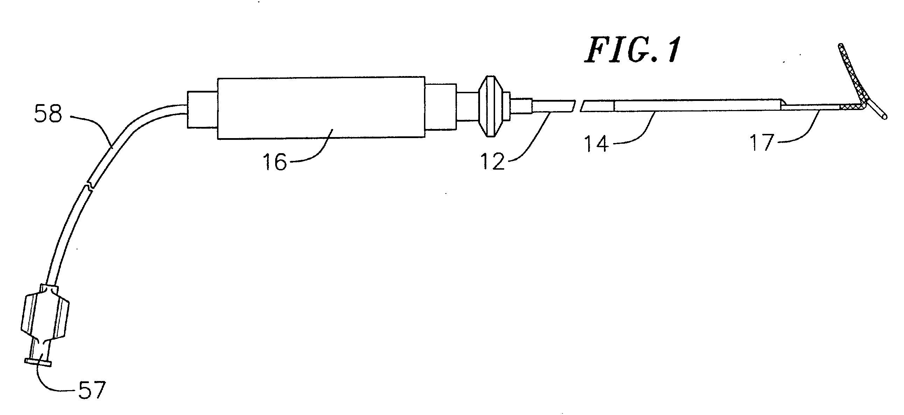

The invention is directed to a catheter having an electrode assembly comprising a braided continuous electrode. The electrode assembly preferably has a curved region that is generally transverse to the axis of the catheter and is irrigated to allow for cooling during ablation. The catheter will now be described in more detail with reference to a particularly preferred embodiment. As shown in FIG 1, the catheter comprises an elongated catheter body **12** having proximal and distal ends, a tip section **14** at the distal end of the catheter body, a control handle 16 at the proximal end of the catheter body, with the electrode assembly **17** mounted at the distal end of the tip section **14**.

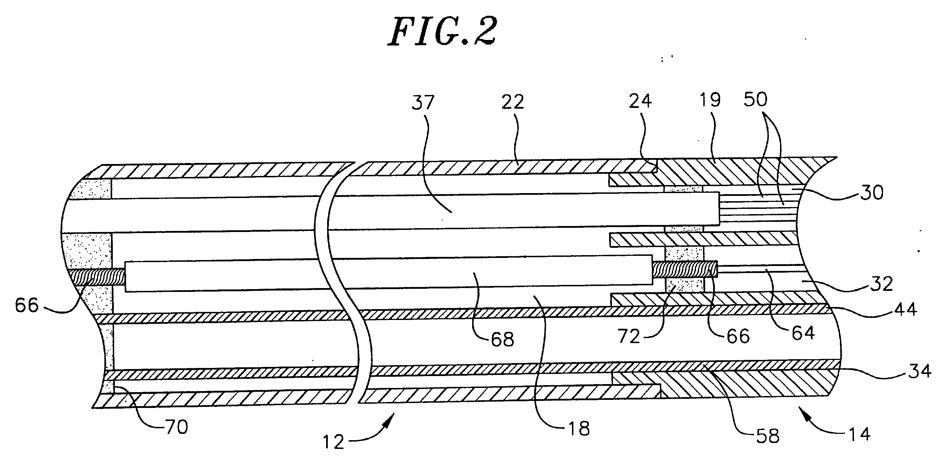

With reference to FIG. 2, the catheter body **12** comprises an elongated tubular construction having a single, axial or central lumen **18**. The catheter body **12** is flexible, i.e., bendable, but substantially non-compressible along its length. The catheter body **12** can be of any suitable construction and made of any suitable material. A presently preferred construction comprises an outer wall **22** made of polyurethane or PEBAX. The outer wall **22** comprises an imbedded braided mesh of stainless steel or the like to increase torsional stiffness of the catheter body **12** so that, when the control handle **16** is rotated, the tip section **14** of the catheter will rotate in a corresponding manner.

The outer diameter of the catheter body **12** is not critical, but is preferably no more than about 8 french, more preferably 7 french. Likewise the thickness of the outer wall **22** is not critical, but is preferably thin enough so that the central lumen **18** can accommodate an infusion tube, a puller wire, lead wires, and any other wires, cables or tubes. If desired, the inner surface of the outer wall **22** is lined with a stiffening tube (not shown) to provide improved torsional stability. A particularly preferred catheter has an outer wall **22** with an outer diameter of from about 0.090 inch to about 0.94 inch and an inner diameter of from about 0.061 inch to about 0.065 inch.

The tip section **14** comprises a short section of tubing **19** having three lumens. The first lumen **30** carries electrode lead wires **50**, the second lumen **32** carries a puller wire **64**, and the third lumen **34** carries an infusion tube **44**. The specific components within the tip section **14** are described in more detail below. The tubing **19** is made of a suitable non-toxic material that is preferably more flexible than the catheter body **12**. A presently preferred material for the tubing **19** is braided polyurethane, i.e., polyurethane with an embedded mesh of braided stainless steel or the like. The size of each lumen is not critical. In a particularly preferred embodiment, the tip section **14** has an outer diameter of about 7 french (.092 inch) and the first lumen **30** and second lumen **32** are generally about the same size, each having a diameter of from about 0.020 inch to about 0.024 inch, preferably 0.022 inch, with the third lumen **34** having a slightly larger diameter of from about 0.032 inch to about 0.038 inch, preferably 0.035 inch.

A preferred means for attaching the catheter body **12** to the tip section **14** is illustrated in FIG. 2. The proximal end of the tip section **14** comprises an outer circumferential notch **24** that receives the inner surface of the outer wall **22** of the catheter body **12**. The tip section **14** and catheter body **12** are attached by glue or the like.

If desired, the catheter body **12** and tip section **14** can be formed of a single, unitary, tubing, rather than being formed of two separate pieces of tubing that are joined together. Such a design might be desirable, for example, where the catheter body **12** and tip section **14** contain the same number of lumens. Accordingly, the term "tip section", as used herein, does not require a separate piece of tubing, but instead designates the distal region of the straight, flexible tubing of the catheter.

If desired, a spacer (not shown) can be located within the catheter body between the distal end of the body and the proximal end of the tip section. The spacer provides a transition in flexibility at the junction of the catheter body and tip section, which allows this junction to bend smoothly without folding or kinking. A catheter having such a spacer is described in U.S. Patent No. 5,964,757, the entire disclosure of which is incorporated herein by reference.

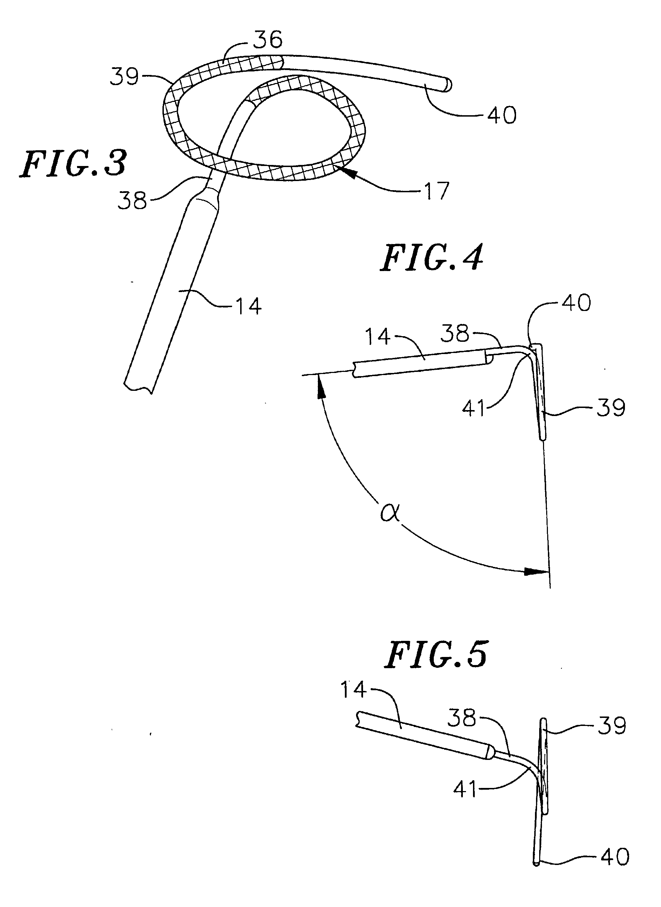

At the distal end of the tip section **14** is a generally circular electrode assembly **17**, as shown in FIGs. 1 and 3 to 5. A preferred electrode assembly **17** comprises a generally straight proximal region **38**, a generally circular main region **39** and a generally straight distal region **40** , as best shown in FIG. 3. The proximal region **38** is mounted on the tip section **14**, as described in more detail below, so that its axis is generally parallel to the axis of the tip section. The proximal region **38** preferably has an exposed length, e.g., not contained within the intermediate section **14**, ranging from about 3 mm to about 12 mm, more preferably about 3 mm to about 8 mm, still more preferably about 5 mm, but can vary as desired. A curved transition region 41 joins the proximal region **38** and the main region **39**.

In the depicted embodiment, the generally circular main region **39** does not form a flat circle, but is very slightly helical, and is generally transverse to the proximal region **38**, tip section **14** and catheter body **12**. The main region **39** generally forms a circle having an outer diameter preferably ranging to about 10 mm to about 30 mm, more preferably about 15 mm to about 25 mm. The transition region **41** of the straight proximal region **38** and generally circular main region **39** is slightly curved and formed such that, when viewed from the side with the proximal region at the top of the circular main region as shown in FIG. 4, the proximal region (along with the tip section **14**) forms an angle α with the curved region preferably ranging from about 75° to about 100°. The main region **39** can curve in a clockwise direction, as shown in FIG. 4, or a counterclockwise direction, as shown in FIG. 5.

The generally circular electrode assembly **17** comprises a continuous electrode **36** along substantially all of the generally circular main region **39**, which preferably has a length ranging from about 15 mm to about 35 mm. Depending on the application, the continuous electrode **36** may cover only a portion of the generally circular main region **39.** The continuous electrode **36** comprises a conductive braided mesh **42** assembled over a flexible plastic tubing **45**. Although the depicted embodiment comprises a substantially circular continuous electrode, the continuous electrode can take any other desired configuration, straight or curved. For some applications, the continuous electrode preferably forms a curve of at least 180 °, and the curve is preferably generally transverse to the axis of the catheter.

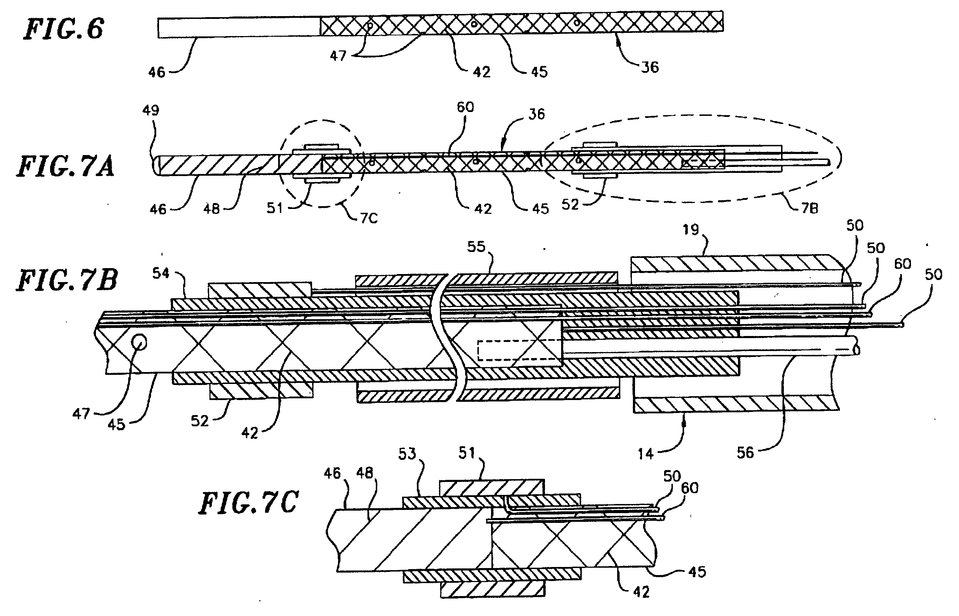

To assemble the preferred continuous electrode **36** of the invention, the braided mesh **42** is formed over the plastic, e.g., polyurethane or PEBAX, tubing **45,** as shown in FIGs. 6, 7A, 7B and 7C. The plastic tubing **45** preferably has an outer diameter ranging from about 0.030 inch to about 0.080 inch, more preferably from about 0.040 inch to about 0.050 inch, with a wall thickness preferably ranging from about 0.005 inch to about 0.020, more preferably about 0.010 inch. The braided mesh **42** comprises interwoven helical members, typically twelve, sixteen or twenty-four interwoven helical members, half extending in one direction and the other half extending in the in the counter direction. The tightness or braid angle of the helical members to a line parallel with the axis of the catheter and intersecting the helical members is not critical, but is preferably about 45 °. The helical members are preferably made of a conductive material having a high modulus of elasticity. Preferred helical members are made of stainless steel wire. Other methods for forming a braided mesh known in the art may be used, for example, methods typically used for forming a metal reinforcing braid within the tubing of the catheter body. During the braiding procedure, preferably the plastic tubing **45** is generally straight and has not been bent to form a generally circular arrangement, as described above. The braided mesh **42** is preferably further secured to the plastic tubing **45** with polyurethane glue or the like.

After the braided mesh **42** is formed over the plastic tubing **45**, the mesh is removed from the distal region **46** of the plastic tubing, as shown in FIG. 6. In a particularly preferred embodiment, the flexible plastic tubing **42** has a length ranging from about 40 mm to about 60 mm, more preferably about 50 mm, where the distal region **46** (which is not covered by the braided mesh) has a length ranging from about 10 mm to about 20 mm, more preferably about 15 mm. Alternatively, the braided mesh **42** can be formed over only a portion of the plastic tubing **45**, although it is typically easier to form the mesh over the entire tubing and thereafter remove a portion of the mesh. Once the plastic tubing **45** is fully assembled to form the generally circular electrode assembly **17**, the distal region **46** of the tubing generally corresponds to the generally straight distal region **40**, and the continuous electrode **36** is formed over substantially all of the generally circular main region **39** and optionally over all or a part of the generally straight proximal region **38**.

Thereafter, irrigation holes **47** are formed in the plastic tubing **45** using a heated needle. It is preferred to form the irrigation holes **47** after the braided mesh **42** has been formed over the plastic tubing **45** so that the glue used to secure the mesh does not block the holes. In the depicted embodiment, the plastic tubing **45** has ten irrigation holes **47** arranged in five pairs that are generally evenly-spaced along the midsection of the plastic tubing. As can be seen in FIG. 6, the irrigation holes **47** in adjacent pairs are offset from each other a distance of about 90° about the circumference of the plastic tubing **45**. By this arrangement, irrigation fluid can be more evenly distributed about the circumference of the continuous electrode **36** (e.g., braided mesh **42**) during use. In a preferred embodiment, the pairs of irrigation holes **47** are longitudinally spaced apart from each other at a distance ranging from about 3 mm to about 5 mm, more preferably about 4 mm. As would be recognized by one skilled in the art, the number and arrangement of the irrigation holes **47** is not critical, but should be adequate to provide the desired amount of irrigation to the continuous electrode **36** depending on the particular application.

After the irrigation holes **47** are formed in the plastic tubing **45**, the distal region **46** of the tubing is preferably modified to provide an atraumatic design that prevents the distal end of the continuous electrode **36** from penetrating tissue during use, as shown in FIG. 7A. In the depicted embodiment, the atraumatic design comprises a tightly wound coil spring **48** mounted within the distal region **46** of the plastic tubing **45** with polyurethane glue **49** or the like. The polyurethane glue **49** also acts to seal the distal end of the plastic tubing **45** (and thus the distal end of the generally circular electrode assembly 17) so that irrigation fluid entering the proximal end of the electrode assembly cannot flow out the distal end. The coil spring **48** is made, for example, of stainless steel, such as the mini guidewire commercially available from Cordis Corporation (Miami, Florida). Additionally, if desired, the distal region **40** of the electrode assembly **17** can be formed, at least in part, of a radiopaque material to aid in the positioning of the electrode assembly **17** under fluoroscopy.

Other alternative atraumatic designs could also be provided. For example, instead of having an elongated atraumatic region, an atraumatic ball (not shown) can be formed on the distal end of the electrode assembly **17**. To form the ball, the distal end of the plastic tubing **45** is covered with a short length of thick non-conductive tubing, made of polyimide, polyurethane or the like. Polyurethane adhesive or the like is applied into and around the edges of the non-conductive tubing to round off the edges of the distal end of the plastic tubing **45**, thereby preventing the distal end of the plastic tubing from damaging the tissue.

The generally straight distal region **40** of the electrode assembly **17** (i.e., the distal region **46** of the plastic tubing **45** housing the coil spring **48**) is preferably sufficiently long to serve as an anchor for introducing the catheter into a guiding sheath, as discussed in more detail below, because the generally circular electrode assembly must be straightened upon introduction into the sheath. Without having the generally straight distal region **40** as an anchor, the generally circular electrode assembly **17** has a tendency to pull out of the guiding sheath upon its introduction into the guiding sheath.

Preferably two ring electrodes **51** and **52** are mounted on the electrode assembly **17**. A distal ring electrode **51** is mounted just distal the continuous electrode **36**, and a proximal ring electrode **52** is mounted just proximal the continuous electrode. During assembly, a distal shrink sleeve **53** is formed over the distal end of the braided mesh **42** and the proximal end of the distal region **46** of the plastic tubing **45**. A proximal shrink sleeve **54** is formed over a proximal region **43** of the plastic tubing **45**. The distal ring electrode **51** is mounted over the distal shrink sleeve **53,** and the proximal ring electrode **52** is mounted over the proximal shrink sleeve **54.** Each ring electrode **51** and **52** is slid over the corresponding shrink sleeve **53** and **54** and fixed in place by glue or the like. The shrink sleeves **53** and **54** electrically isolate the ring electrodes **51** and **52** from the continuous electrode **36.** The ring electrodes **51** and **52** can be made of any suitable solid conductive material, such as platinum or gold, and are preferably machined from platinum-iridium bar (90% platinum/10% iridium). Alternatively, the ring electrodes can be formed by coating the shrink sleeves **53** and **54** with an electrically conducting material, like platinum, gold and/or iridium. The coating can be applied using sputtering, ion beam deposition or an equivalent technique.

The ring electrodes **51** and **52** are each connected to a separate electrode lead wire **50**. The lead wires **50** extend through the first lumen **30** of tip section **14**, the central lumen **18** of the catheter body **12**, and the control handle **16**, and terminate at their proximal end in an input jack (not shown) mounted in the handle that may be plugged into an appropriate source of ablation energy, e.g., radio frequency energy, and/or to a monitor. The portion of the lead wires **50** extending through the central lumen **18** of the catheter body **12**, control handle **16** and proximal end of the tip section **14** may be enclosed within a protective sheath **37**, which can be made of any suitable material, preferably polyimide. The protective sheath **37** is preferably anchored at its distal end to the proximal end of the tip section **14** by gluing it in the first lumen **30** with polyurethane glue or the like.

The lead wires **50** are attached to the ring electrodes **51** and **52** by any conventional technique. Connection of a lead wire **50** to the distal ring electrode **51** is preferably accomplished by first making a small hole through the plastic tubing **45** and corresponding shrink sleeve **53**. Such a hole can be created, for example, by inserting a needle through the tubing **45** and shrink sleeve **53** and heating the needle sufficiently to form a permanent hole. A lead wire **50** is then drawn through the hole by using a microhook or the like. The ends of the lead wire **50** are then stripped of any coating and soldered or welded to the underside of the ring electrode **51**, which is then slid into position over the hole and fixed in place with polyurethane glue or the like. The lead wire **50** for the proximal ring electrode **52** preferably extends outside the plastic tubing **45** and proximal shrink sleeve **54** and is soldered to the underside of the proximal ring electrode. An outer proximal shrink sleeve **55** is then provided over the lead wire **50** connected to the proximal ring electrode to protect that lead wire.

Another lead wire **50** is electrically connected to the continuous electrode **36**. Preferably the lead wire **50** is soldered to the proximal end of the braided mesh **42** prior to placement of the proximal shrink sleeve **54**. The proximal shrink sleeve **42** thus acts to protect the lead wire **50** connected to the continuous electrode **36**. Any other suitable method for attaching a lead wire to the continuous electrode can also be used.

A short irrigation tube **56** fluidly connects the third lumen **34** of the tip section **14** to the interior of the electrode assembly **17**. The irrigation tube **56** is preferably made of polyimide, but can be made of any other suitable biocompatible material. The irrigation tube **56** provides a means for introducing irrigation fluid into the electrode assembly **17**. Irrigation fluid is introduced into the third lumen **34** and irrigation tube **56** by means of an infusion tube **58**, which has its distal end mounted in the proximal end of the third lumen **34** of the tip section **14** and which extends through the catheter body **12**, out the proximal end of the control handle **16**, and terminates in a luer hub **57** or the like at a location proximal to the control handle. In an alternative arrangement, a single lumen side arm (not shown) is fluidly connected to the central lumen **18** near the proximal end of the catheter body **12**, as described in more detail in U.S. Patent No. 6,120,476, the entire disclosure of which is incorporated herein by reference. Other means known in the art for introducing fluid into the electrode assembly could also be provided.

Preferably the generally circular shape of the electrode assembly **17** is maintained with a support member **60** that is mounted in at least a portion of the plastic tubing **45** of the electrode assembly. The support member **60** is preferably made of a material having shape-memory, i.e., that can be straightened or bent out of its original shape upon exertion of a force and is capable of substantially returning to its original shape upon removal of the force. A particularly preferred material for the support member **60** is a nickel/titanium alloy. Such alloys typically comprise about 55% nickel and 45% titanium, but may comprise from about 54% to about 57% nickel with the balance being titanium. A preferred nickel/titanium alloy is nitinol, which has excellent shape memory, together with ductility, strength, corrosion resistance, electrical resistivity and temperature stability.

In the depicted embodiment, the support member **60** has a proximal end mounted in the tip section **14** and a distal end soldered to the proximal end of the coil spring **48.** Accordingly, the support member extends entirely through the generally circular main region **39** so as to define the shape of this region. By having the proximal end of the support member **60** mounted in the tip section **14,** the support member acts to maintain the electrode assembly **17** on the tip section. Having a support member **60** made of a material with shape memory allows the electrode assembly **17** to be straightened when being introduced into the heart through a guiding sheath and then return to its curved formation after the guiding sheath is removed in the heart, as described in more detail below.

If desired, a temperature sensing means (not shown) can be provided for the electrode assembly **17**. Any conventional temperature sensing means, e.g., a thermocouple or thermistor, may be used. One preferred temperature sensing means comprises a thermocouple formed by a wire pair. One wire of the wire pair is a copper wire, e.g., a number **38** copper wire. The other wire of the wire pair is a constantan wire, which gives support and strength to the wire pair. The wires and can extend through the first lumen **30** in the tip section **14** along with the lead wires **50**. Within the catheter body **12** the wires and can extend through the protective sheath **37**, also with the lead wires **50**. The wires then extend out through the control handle **16** and to the connector (not shown) in the handle, which is connectable to a temperature monitor (not shown).

A puller wire **64** is provided for deflection of the tip section **14**. The puller wire **64** extends through the catheter body **12**, is anchored at its proximal end to the control handle **16**, and is anchored at its distal end to the tip section **14**, as described in more detail below. The puller wire **64** is made of any suitable metal, such as stainless steel or Nitinol, and is preferably coated with Teflon® or the like. The coating imparts lubricity to the puller wire **64**. The puller wire **64** preferably has a diameter ranging from about 0.006 to about 0.010 inch.

A compression coil **66** is situated within the catheter body **12** in surrounding relation to the puller wire **64**. The compression coil **66** extends from the proximal end of the catheter body **12** to the proximal end of the tip section **14.** The compression coil **66** is made of any suitable metal, preferably stainless steel. The compression coil **66** is tightly wound on itself to provide flexibility, i.e., bending, but to resist compression. The inner diameter of the compression coil **66** is preferably slightly larger than the diameter of the puller wire **64**. The Teflon® coating on the puller wire **64** allows it to slide freely within the compression coil **66**. If desired, particularly if the lead wires **50** are not enclosed by a protective sheath **62**, the outer surface of the compression coil **66** is covered by a flexible, non-conductive sheath **68,** e.g., made of polyimide tubing, to prevent contact between the compression coil and any other wires within the catheter body **12**.

The compression coil **66** is anchored at its proximal end to the outer wall **22** of the catheter body **12** by proximal glue joint **70** and at its distal end to the tip section **14** by distal glue joint **72**. Both glue joints **70** and **72** preferably comprise polyurethane glue or the like. The glue may be applied by means of a syringe or the like through a hole made between the outer surface of the catheter body **12** and the central lumen **18**. Such a hole may be formed, for example, by a needle or the like that punctures the outer wall **22** of the catheter body **12** which is heated sufficiently to form a permanent hole. The glue is then introduced through the hole to the outer surface of the compression coil **66** and wicks around the outer circumference to form a glue joint about the entire circumference of the compression coil **66**.

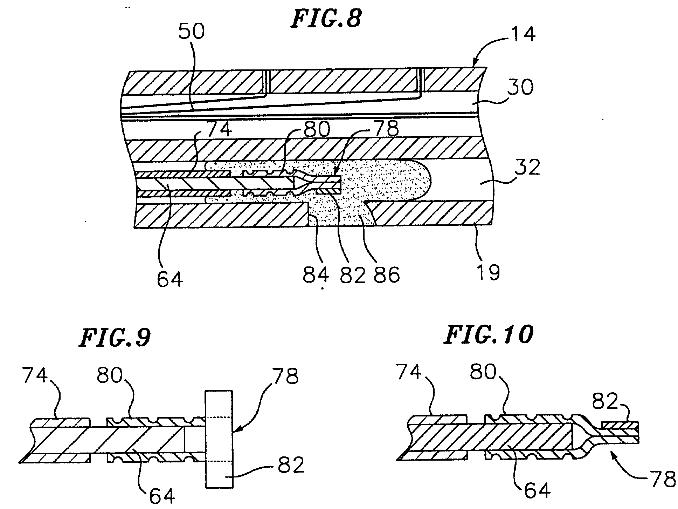

The puller wire **64** extends into the second lumen **32** of the tip section **14**. Preferably the puller wire **64** is anchored at its distal end to the side of the tip section **14**, as shown in FIGs. 8 to **10**. A T-shaped anchor **78** is formed, which comprises a short piece of tubular stainless steel **80**, e.g., hypodermic stock, which is fitted over the distal end of the puller wire **64** and crimped to fixedly secure it to the puller wire. The distal end of the tubular stainless steel **80** is fixedly attached, e.g., by welding, to a stainless steel cross-piece **82** such as stainless steel ribbon or the like. The cross-piece **82** sits in a notch **84** in a wall of the flexible tubing **19** that extends into the second lumen **32** of the tip section **14**. The stainless steel cross-piece **82** is larger than the opening and, therefore, cannot be pulled through the opening. The portion of the notch **84** not filled by the cross-piece **82** is filled with glue **86** or the like, preferably a polyurethane glue, which is harder than the material of the flexible tubing **19**. Rough edges, if any, of the cross-piece **82** are polished to provide a smooth, continuous surface with the outer surface of the flexible tubing **19**. Within the second lumen **32** of the tip section **14**, the puller wire **64** extends through a plastic, preferably Teflon®, puller wire sheath **74**, which prevents the puller wire **64** from cutting into the wall of the tip section **14** when the tip section is deflected. Any other suitable technique for anchoring the puller wire **64** in the tip section **14** can also be used.

Longitudinal movement of the puller wire **64** relative to the catheter body **12**, which results in deflection of the tip section **14**, is accomplished by suitable manipulation of the control handle **16**. Examples of suitable control handles for use in the present invention are disclosed, for example, in U.S. Patent Nos. Re 34,502 and 5,897,529, the entire disclosures of which are incorporated herein by reference.

In use, a suitable guiding sheath is inserted into the patient. An example of a suitable guiding sheath for use in connection with the present invention is the Preface™ Braiding Guiding Sheath, commercially available from Biosense Webster (Diamond Bar, California). The distal end of the sheath is guided into one of the atria. A catheter in accordance with the present invention is fed through the guiding sheath until its distal end extends out of the distal end of the guiding sheath. As the catheter is fed through the guiding sheath, the curved electrode assembly can be straightened to fit through the sheath, and it will return to its original shape upon removal of the sheath. The continuous electrode is then used to form continuous linear lesions by ablation. As used herein, a linear lesion refers to any lesion, whether curved or straight.

For treating atrial fibrillation, the linear lesion is formed between two anatomical structures in the heart and is sufficient to block a wavelet, i.e., forms a boundary for the wavelet. Anatomical structures, referred to as "atrial trigger spots", are those regions in the heart having limited or no electrical conductivity and are described in Haissaguerre et al., "Spontaneous Initiation of Atrial Fibrillation by Ectopic Beats Originating in the Pulmonary Veins", New England Journal of Medicine, 339:659-666 (Sept. 3, 1998), the disclosure of which is incorporated herein by reference. The linear lesions typically have a length of from about 1 cm to about 4 cm, but can be longer or shorter as necessary for a particular procedure.

During ablation, irrigation fluid may be introduced through the irrigation tube and infusion tube, into the flexible tubing of the mapping assembly, and out through the irrigation holes. The fluid acts to cool the electrode and surrounding tissue during the ablation procedure and to displace blood away from the electrode. This allows more energy to be delivered safely into the tissue to form deeper lesions and to reduce the possibility of blood coagulation. The fluid introduced through the catheter is preferably a biologically compatible fluid, such as saline.

The rate of fluid flow through the catheter may be controlled by any suitable fluid infusion pump or by pressure. A suitable infusion pump is the FLOGARD™ pump, available from Baxter. The rate of fluid flow through the catheter preferably ranges from about 0.5 ml/min to about 30 ml/min, more preferably from about 5 ml/min to about 15 ml/min. Preferably the fluid is maintained at about room temperature.

If desired, two or more puller wires can be provided to enhance the ability to manipulate the tip section. In such an embodiment, a second puller wire and a surrounding second compression coil extend through the catheter body and into an additional off-axis lumen in the tip section. The first puller wire is preferably anchored proximal to the anchor location of the second puller wire. Suitable designs of catheters having two or more puller wires, including suitable control handles for such embodiments, are described, for example, in U.S. Patent Nos. 6,123,699,6,171,277, and 6,183,463, and allowed U.S. Patent Application No.09/157,055, filed September 18, 1998, the disclosures of which are incorporated herein by reference.

The preceding description has been presented with reference to presently preferred embodiments of the invention. Workers skilled in the art and technology to which this invention pertains will appreciate that alterations and changes in the described structure may be practiced without meaningfully departing from the principal, spirit and scope of this invention.

Accordingly, the foregoing description should not be read as pertaining only to the precise structures described and illustrated in the accompanying drawings, but rather should be read consistent with and as support to the following claims which are to have their fullest and fair scope.

## Claims

1. A catheter for ablating tissue comprising an elongated flexible catheter body having a continuous electrode comprising a braided conductive mesh surrounding a flexible plastic tubing.

2. A catheter according to claim 1, further comprising an electrode lead wire having a distal end electrically connected to the continuous electrode and a proximal end electrically connected to a source of ablation energy.

3. A catheter according to claim 1, wherein the flexible tubing has an outer wall with a plurality of irrigation openings extending therethrough, and wherein the catheter further comprises means for introducing irrigation fluid into the flexible tubing, whereby, in use, fluid can pass out of the continuous electrode through the irrigation holes in the flexible tubing.

4. A catheter for ablating tissue comprising:
a catheter body having an outer wall, proximal and distal ends, and at least one lumen extending therethrough;
a tip section comprising a segment of flexible tubing having proximal and distal ends and at least one lumen therethrough, the proximal end of the tip section being fixedly attached to the distal end of the catheter body;
an electrode assembly at the distal end of the tip section, the electrode assembly comprising a flexible plastic tubing and a continuous electrode formed of a braided conductive mesh surrounding the flexible plastic tubing; and
an electrode lead wire electrically connecting the continuous electrode to a source of ablation energy.

5. A catheter according to claim 4, wherein the electrode assembly further comprises a support member having shape memory extending within a lumen of the flexible plastic tubing.

6. A catheter according to claim 4, wherein the flexible plastic tubing has an outer wall, at least one lumen extending through the flexible plastic tubing, and a plurality of irrigation holes extending through the outer wall of the flexible plastic tubing, and wherein the catheter further comprises means for introducing an irrigation fluid into the lumen of the flexible plastic tubing of the electrode assembly, whereby, in use, fluid can pass out of the electrode assembly through the irrigation holes.

7. A catheter according to claim 4, wherein the electrode assembly comprises a curved region over which the continuous electrode extends.

8. A catheter according to claim 7, wherein the curved region is generally transverse to the axis of the catheter body.

9. A catheter according to any one of claims 1 to 8, wherein the continuous electrode has a length of at least about 15 mm.

10. A catheter for ablating tissue comprising:
a catheter body having an outer wall, proximal and distal ends, and at least one lumen extending therethrough;
a tip section comprising a segment of flexible tubing having proximal and distal ends and at least one lumen therethrough, the proximal end of the tip section being fixedly attached to the distal end of the catheter body;
an electrode assembly at the distal end of the tip section, the electrode assembly comprising a flexible plastic tubing having an outer wall with a plurality of irrigation holes extending therethrough, a generally circular main region that is generally transverse to the axis of the catheter body, a continuous electrode formed of braided conductive mesh surrounding the flexible plastic tubing and extending over substantially all of the generally circular main region, and a support member having shape memory extending within a lumen of the flexible plastic tubing;
an electrode lead wire electrically connecting the continuous electrode to a source of ablation energy; and
means for introducing an irrigation fluid into a lumen of the flexible plastic tubing of the electrode assembly so that the fluid can pass out of the electrode assembly through the irrigation holes.
